# EUROPÄISCHE PATENTSCHRIFT

(11) **EP 1 282 446 B1**
(45) Veröffentlichungstag und Bekanntmachung des Hinweises auf die Patenterteilung: **05.12.2007**
(21) Anmeldenummer: 01940156.1
(22) Anmeldetag: 14.04.2001
(51) Int. Cl.: A61K 47/24, A61K 9/12, A61K 9/127, A61K 9/48, A61K 8/04, A61K 8/55, A61K 8/58, A61Q 19/00

(54) **PHARMAZEUTISCHE UND/ODER KOSMETISCHE ZUBEREITUNG ENTHALTEND EIN ORGANOSILOXAN UND EIN PHOSPHOLIPID**
PHARMACEUTICAL AND/OR COSMETIC COMPOSITION CONTAINING AN ORGANOSILOXANE AND A PHOSPHOLIPID
COMPOSITION PHARMACEUTIQUE ET/OU COSMETIQUE CONTENANT UN ORGANOSILOXANE ET UN PHOSPHOLIPIDE

(30) Priorität: 19.05.2000 DE 10024413
(43) Veröffentlichungstag der Anmeldung: 12.02.2003
(60) Teilanmeldung: 07004211.4
(73) Patentinhaber: MIKA Pharma Gesellschaft für die Entwicklung und Vermarktung pharmazeutischer Produkte mbH, 67117 Limburgerhof (DE)
(72) Erfinder: PIOTROWIAK, Ralf, 32545 Bad Oeynhausen (DE); SEIGFRIED, Bernd, G., 67117 Limburgerhof (DE)
(74) Vertreter: Lau-Loskill, Philipp
(86) Internationale Anmeldenummer: PCT/DE2001/001483
(87) Internationale Veröffentlichungsnummer: WO 2001/087344

(56) Entgegenhaltungen:
- EP-A- 0 834 312
- WO-A-99/38483
- FR-A- 2 777 179
- FR-A- 2 777 181
- US-A- 5 582 815
- US-A- 5 660 853
- PATENT ABSTRACTS OF JAPAN vol. 018, no. 437 (C-1238), 16. August 1994 (1994-08-16) -& JP 06 135816 A (EARTH CHEM CORP LTD), 17. Mai 1994 (1994-05-17)
- BALSAM M.S. ET AL: 'Cosmetics Science and Technology', 1979, WILEY INTERSCIENCE, NEW YORK
- LIST P.H. ET AL: 'Arzneiformenlehre', WISSENSCHAFTLICHE VERLAGSGESELLSCHAFT MBH, STUTTGART

## Beschreibung

Die vorliegende Erfindung betrifft eine pharmazeutische und/oder kosmetische Zubereitung zur Anwendung beim Menschen, beim Tier oder bei Pflanzen mit den Merkmalen des Oberbegriffs des Patentanspruchs 1.

Kosmetische und/oder pharmazeutische Zubereitungen der eingangs genannten Art sind in unterschiedlichen Darreichungsformen und Formulierungen bekannt. Hierbei weisen diese bekannten Zubereitungen mindestens einen entsprechenden pharmazeutischen und/oder kosmetischen Wirkstoff auf, während andere Inhaltsstoffe der bekannten Zubereitungen dazu dienen, bestimmte, erwünschte Eigenschaften des Wirkstoffes selbst oder der Zubereitung, so zum Beispiel eine spezielle Darreichungsform, zu ermöglichen.

Auch sind pharmazeutischen Zubereitungen zur Anwendung beim Menschen bekannt, wobei diese bekannten Zubereitungen neben mindestens einem pharmazeutischen Wirkstoff desweiteren mindestens eine siliciumorganische Verbindung auf der Basis eines oligomeren und/oder polymeren Diorganosiloxans aufweisen. So beschreibt die US 5,582,815 eine therapeutische oder kosmetische Zubereitung, die topisch in Form eines Aerosols appliziert wird und die neben einem therapeutischen oder kosmetischen Wirkstoff, vorzugsweise neben einem Mittel gegen Pilzbefall, eine hohe Konzentration eines Polydiorganosiloxans aufweist.

Vielfach besteht jedoch das Problem, daß entsprechende pharmazeutische und/oder kosmetische Wirkstoffe nicht oder nur sehr bedingt in dem jeweils eingesetzten Diorganosiloxan löslich oder dispergierbar sind, so daß die Herstellung einer gegen Entmischung stabilen entsprechenden pharmazeutischen und/oder kosmetischen Zubereitung erhebliche Probleme bereitet.

Darüber hinaus ist es, so zum Beispiel aus "List, Müller und Nürnberg, Arzneiformenlehre - ein Lehrbuch für Pharmazeuten - Wissenschaftliche Verlagsgesellschaft mbH, Stuttgart, 1985", bekannt, für die Herstellung von pharmazeutischen Produkten phospholipidische Emulgatoren zu verwenden, um Wirkstoffe stabil in Wasser als Lösungsmittels zu emulgieren. Diese allgemeine Aussage wird für einen Wirkstoff auf der Basis von Diclofenac in Wasser oder in wasserhaltigen Lösungsmittelgemischen, das bzw. die zusätzlich einen phospholipidischen Lösungsvermittler enthält bzw. enthalten, durch die EP-A 0 834 312 konkretisiert.

Der vorliegenden Erfindung liegt die Aufgabe zugrunde, eine pharmazeutische und/oder kosmetische Zubereitung zur Anwendung beim Menschen, beim Tier oder bei der Pflanze zur Verfügung zu stellen, die eine besonders hohe Stabilität besitzt und in der der mindestens eine Wirkstoff besonders gut gelöst bzw. dispergiert ist.

Diese Aufgabe wird erfindungsgemäß durch eine pharmazeutische und/oder kosmetische Zubereitung der zuvor genannten Art mit dem kennzeichnenden Merkmal des Patentanspruchs 1 gelöst.

Die erfindungsgemäße pharmazeutische und/oder kosmetische Zubereitung zur Anwendung beim Menschen, beim Tier oder bei Pflanzen weist mindestens einen pharmazeutischen bzw. kosmetischen Wirkstoff sowie mindestens eine siliciumorganische Verbindung auf der Basis eines Diorganosiloxanes auf, wobei die erfindungsgemäße Zubereitung desweiteren mindestens ein Phospholipid und als siliciumorganische Verbindung ein Hexamethyldisiloxan enthält. Mit anderen Worten unterscheidet sich somit die erfindungsgemäße Zubereitung von der zuvor in Verbindung mit der US 5,582,815 abgehandelten bekannten Zubereitung dahingehend, daß zusätzlich in der erfindungsgemäßen Zubereitung mindestens ein Phospholipid vorhanden ist.

Der bei der erfindungsgemäßen Zubereitung verwendete Begriff pharmazeutische Wirkstoff soll auch solche Wirkstoffe abdecken, die bei Pflanzen eine bestimmte biologische Wirksamkeit besitzen, wie dies nachfolgend noch erläutert ist.

Die zuvor beschriebene erfindungsgemäße Zubereitung weist eine Reihe von Vorteilen auf. So ist zunächst festzuhalten, daß sie eine ausgezeichnete Stabilität besitzt, so daß selbst bei einer längeren Lagerung, insbesondere auch bei erhöhten Temperaturen, keine Entmischung der Inhaltsstoffe, kein Agglomerieren des Wirkstoffes bzw. der Wirkstoffe und auch kein unerwünschter chemischer Abbau des Wirkstoffes bzw. der Wirkstoffe stattfindet. Dies ist um so erstaunlicher, zumal bekannt ist, daß Phospholipide, insbesondere auch Phosphatidylcholin-haltige Phospholipide, bei einer Lagerung zu einem unerwünschten chemischen Abbau neigen, wobei derartige Abbauprodukte dann die Gefahr beinhalten können, daß hierdurch der chemische Abbau des Wirkstoffes bzw. der Wirkstoffe katalysiert wird. Dies tritt jedoch, wie bereits vorstehend beschrieben, überraschenderweise in der erfindungsgemäßen Zusammensetzung nicht auf.

Desweiteren konnte festgestellt werden, daß durch das Vorhandensein des mindestens einen Phospholipids in der erfindungsgemäßen Zubereitung die Löslichkeit bzw. die Dispergierbarkeit des Wirkstoffes bzw. der Wirkstoffe in vielen Fällen erst ermöglicht oder zumindestens erheblich verbessert wird, so daß hierdurch im Vergleich zu solchen bekannten Zubereitungen, die lediglich neben dem Wirkstoff noch die zuvor genannte siliciumorganische Verbindung aufweisen, die Möglichkeiten zur Herstellung von Formulierungen auf eine Vielzahl von Wirkstoffen erweitert wird.

So konnte beispielsweise festgestellt werden, daß die Löslichkeit des Wirkstoffes Clotrimazol in Hexamethyldisiloxan allein kleiner als 0,025 Gew.% ist, während sich dieser Wirkstoff in einer Zubereitung, die 10 Gew.%. Phospholipid und 90 Gew.% Hexamethyldisiloxan enthält, mit einer 10-fach höheren Konzentration löst, wobei das hierfür verwendete Phospholipid 25 Gew.% Ethanol und 75 Gew.% Phospholipid aufwies, das 76 ± 3 Gew.% Phosphatidylcholin, 3 ± 3 Gew.% Lysophosphatidylcholin, bis 8 Gew.% Phosphatidsäure, bis 4 Gew.% Phosphatidylethanolamin und maximal 9 Gew.% sonstiger phospholipidische und nicht-phospholipidische Bestandteile, wie insbesondere Öle, Fette und/oder Triglyceride, enthielt.

Außerdem bewirkt der Zusatz des mindestens einen Phospholipids in der erfindungsgemäßen Zubereitung, daß innerhalb von kürzester Zeit die lokal äußerlich aufgetragene Zubereitung unmittelbar in die Haut des Menschen oder Tiers bzw. in die äußeren Pflanzenschichten der hiermit behandelten Pflanzen einzieht, so daß ein unerwünschtes Abreiben einer derartig topisch aufgetragenen erfindungsgemäßen Zubereitung oder ein Abwaschen derselben nicht auftreten kann. Bei einer derartigen Applikation bildet sich innerhalb von wenigen Sekunden zunächst eine gelartige Formulierung auf der Haut aus, so daß die ursprünglich flüssig aufgetragene Zubereitung aufgrund dieser Konsistenzumwandlung in der aufgetragenen Zone fixiert wird und hiernach rasch in das Körperinnere einzieht.

Ferner wirkt die erfindungsgemäße Zubereitung rückfettend, so daß dementsprechend hiermit behandelte äußere Körperbereiche entsprechend gepflegt werden, mit der Folge, daß eine entsprechende Heilung oder Linderung beschleunigt herbeigeführt wird. Hautirritationen, wie sie insbesondere bei empfindlichen Anwendern bei Mensch und Tier bei der Verwendung von bekannten Zubereitungen, die kein Phospholipid enthalten, beobachtet werden konnten, treten bei der Anwendung der erfindungsgemäßen Zubereitung nicht auf. Von daher verursacht die erfindungsgemäße Zubereitung, in der maximal 14 Gew.% Alkohol enthalten ist, bei einer topischen Applikation auf empfindliche oder vorgeschädigte Haut auch keine zusätzlichen Schmerzen, wie das üblicherweise bei den gängigen topischen Formulierungen, die mehr als 14,5 Gew.% Alkohol enthalten, der Fall ist, so daß der entsprechende Anwender die hieraus resultierende Anwendungsbarriere bei der erfindungsgemäßen Zubereitung nicht überwinden muß, wodurch eine ständige und reproduzierbare Anwendung, die letztendlich den Heilungserfolg sicherstellt, gewährleistet wird. Desweiteren wird durch den mindestens einen Phospholipid-Zusatz in der erfindungsgemäßen Zubereitung bei einer Vielzahl von unterschiedlich strukturierten Wirkstoffen sichergestellt, daß transparente Lösungen bzw. transparente feindisperse Systeme entstehen, die einerseits steril filtrierbar sind und andererseits sehr leicht aufgrund ihrer Transparenz auf Fremdpartikel kontrolliert werden können. Diese Kontrollmöglichkeit ist insbesondere dann sehr wichtig, wenn die erfindungsgemäße Zubereitung so formuliert wird, daß sie als Infusion oder Injektion zu applizieren ist.

Insbesondere für solche Ausführungsformen der erfindungsgemäßen Zubereitung, die topisch beim Menschen oder beim Tier angewendet werden, ist als besonderer Vorteil festzuhalten, daß sie sehr schnell, vorzugsweise zwischen 2 Sekunden und 30 Sekunden, nach dem Auftragen auf die jeweiligen Körperbereiche nach innen hin einziehen, so daß auf der Oberfläche keine Rückstände, insbesondere auch keine schmierenden Rückstände, verbleiben. Von daher wird bei dieser Ausgestaltung der erfindungsgemäßen Zubereitung im hohem Maße sichergestellt, daß die topisch applizierte Menge auch tatsächlich innerhalb von kürzester Zeit in den Körper gelangt und daß ein Verschmutzen der Verkleidung ausgeschlossen ist, wodurch eine hohe Anwenderfreundlichkeit der erfindungsgemäßen Zubereitung zur Verfügung gestellt wird.

Als weiterer Vorteil der erfindungsgemäßen Zubereitung, die die zuvor genannte siliciumorganische Verbindung enthält, ist festzuhalten, daß eine derartige siliciumorganische Verbindung aufgrund ihres schnellen Verdunstens eine Kühlwirkung im Bereich der aufgetragenen erfindungsgemäßen Zubereitung hervorruft, was insbesondere dann als sehr angenehm empfunden wird, wenn die erfindungsgemäße Zubereitung zur Behandlung von entzündlichen Zuständen oder Prellungen oder zur Behandlung von sonstigen Sportverletzungen topisch angewandt wird.

Bezüglich des in der erfindungsgemäßen Zubereitung enthaltenen mindestens einen Phospholipids ist festzuhalten, daß es sich hierbei vorzugsweise um ein solches Phospholipid bzw. Phospholipidgemisch handelt, das aus natürlichen Ausgangsstoffen, so insbesondere Soja, Saflor, Baumwollsaaten, Sonnenblumen und/oder Ei, isoliert ist, wobei derartige Phospholipide vorzugsweise als ölfreie Phospholipide verwendet werden.

Besonders gute Ergebnisse bezüglich der Stabilität und der eingangs bei der erfindungsgemäßen Zubereitung beschriebenen vorteilhaften Eigenschaften, so beispielsweise dem hohe Löse- bzw. Dispergiervermögen, der sterilen Filtrierbarkeit, der leichten Kontrollmöglichkeit auf Fremdpartikel und der rückfettenden Wirkung, besitzen solche Ausgestaltungen der erfindungsgemäßen Zubereitung, in denen als Phospholipid mindestens ein an Phosphatidylcholin (1,2-Diacylglycero-3-Phosphatidylcholin) reiches Phospholipid vorhanden ist, wobei vorzugsweise solche Phospholipide ausgewählte werden, die mehr als 70 Gew.%, insbesondere mindestens 80 Gew.% und vorzugsweise mehr als 90 Gew.% Phosphatidylcholin, bezogen auf das Gesamtgewicht des jeweils verwendeten Phospholipids, enthalten.

Wenn bei einem derartigen, an Phosphatidylcholin reichem Phospholipid bzw. Phospholipidgemisch zusätzlich noch dessen Acylreste in Position 1 und in Position 2 zu 10 bis 15 Gew.% aus dem Palmitinsäurerest, zu 1,4 bis 4 Gew.% aus dem Stearinsäurerest, zu 3 bis 10 Gew.% aus dem Ölsäurerest, zu 61 bis 71 Gew.% aus dem Linolsäurerest und zu 3 bis 7 Gew.% aus dem Linolensäurerest, bezogen auf die Summe aller Acylreste in Position 1 und 2, bestehen, verleiht ein derartiges Phosphatidylcholin in Verbindung mit der zuvor beschriebenen siliciumorganischen Verbindung der erfindungsgemäßen Zubereitung eine hohe chemische Stabilität, so daß ein unerwünschter Abbau des Wirkstoffes bzw. des Wirkstoffgemisches selbst bei einer mehrmonatigen Lagerung unter extremen Bedingungen nicht oder nur im völlig untergeordnetem Maße stattfindet.

Bei einer anderen Ausgestaltung der erfindungsgemäßen Zubereitung weist diese als Phospholipid ein hydriertes Phospholipid, vorzugsweise ein hydriertes Phosphatidylcholin, auf. Derartige hydrierte Phospholipide erleichtern desweiteren die Herstellung von transparenten Zubereitungen, so daß hier nur ein kurzes Vermischen der Inhaltsstoffe der erfindungsgemäßen Zubereitung erforderlich ist, um entsprechend steril filtrierbare und transparente Lösungen bzw. Feindispersionen des Wirkstoffes zu erstellen, die dann entsprechend, ggf. nach Sterilfiltration durch ein 0,2 *µ*m Filter und/oder nach zusätzlicher Sterilisation, dann auch sicher durch Injektion oder Infusion dargereicht werden können.

Das Verhältnis der siliciumorganischen Verbindung zu dem mindestens einen Phospholipid hängt allgemein insbesondere von der gewünschten Darreichungsform, der ausgewählten siliciumorganischen Verbindung, dem jeweils verwendeten Phospholipid und dem mindestens einen, in der erfindungsgemäßen Zubereitung enthaltenen pharmazeutischen und/oder kosmetischen Wirkstoff ab. Hervorragende Löse- bzw. Dispergiereigenschaften bei ausgezeichneter Stabilität lassen sich dadurch erreichen, daß die erfindungsgemäße Zubereitung die siliciumorganische Verbindung in einer Konzentration zwischen 5 Gew.% und 96 Gew.% und das mindestens eine Phospholipid in einer Konzentration zwischen 2 Gew.% und 30 Gew.%, jeweils bezogen auf das Gesamtgewicht der Zubereitung, enthalten. Besonders geeignet ist es, wenn die erfindungsgemäße Zubereitung zwischen 20 Gew.% und 85 Gew.% der siliciumorganischen Verbindung und zwischen 5 Gew.% und 20 Gew.% des mindestens einen Phospholipids enthält.

Durch Variation und Abstimmung der zuvor beschriebenen siliciumorganischen Verbindung und des zuvor beschriebenen mindestens einen Phospholipids in Kombination mit Wasser lassen sich auch Ausführungsformen der erfindungsgemäßen Zubereitung formulieren, deren Wassergehalt zwischen 0 Gew. % und 5 Gew. % variiert und bei denen die in der Zubereitung enthaltene siliciumorganische Verbindung und das Phospholipid derart miteinander vermischt sind, daß die Zubereitung bei Kontakt mit Wasser spontan Liposome und/oder Mizellen ausbildet. Hierbei zeigte sich, daß überraschenderweise bereits die geringe, in der Haut und insbesondere in den oberen Hautschichten natürlicherweise vorkommende Wasserkonzentration ausreicht, um auf der Hautoberfläche spontan diese Liposome und/oder Mizellen auszubilden, so daß eine derartige Weiterbildung der erfindungsgemäßen Zubereitung insbesondere für die topische Anwendung bevorzugt wird.

Vorzugsweise werden bei der zuvor beschriebenen Ausgestaltung der erfindungsgemäßen Zubereitung die siliciumorganische Verbindung, das Phospholipid und das Wasser derart aufeinander abgestimmt, daß die sich durch Zugabe von Wasser spontan bildenden Liposome bzw. Mizellen eine mittlere Teilchengröße zwischen 50 nm und 4.000 nm, insbesondere zwischen 250 nm und 1.500 nm, aufweisen, so daß derartige Liposome bzw. Mizellen besonders geeignet sind, die in der erfindungsgemäßen Zubereitung enthaltenen Wirkstoffe bei einer topischen Applikation schnell und vollständig in das Innere des menschlichen oder tierischen Körpers oder in das Innere der Pflanze zu transportieren. Hierdurch wird insbesondere auch sichergestellt, daß der jeweilige Wirkstoff schnell und zerstörungsfrei dort hingelangt, wo er die diesbezügliche kosmetische bzw. pharmazeutische Wirksamkeit zeigen soll.

Abhängig von der jeweiligen Verwendung der erfindungsgemäßen Zubereitung und deren Darreichungsform weist die erfindungsgemäße Zubereitung neben der einen siliciumorganischen Verbindung der eingangs beschriebenen Art und dem Phospholipid ggf. desweiteren Wasser, Alkohol, Antioxidantien, nicht phospholipidische Emulgatoren, pharmazeutische Wirkstoffe, kosmetische Wirkstoffe und/oder weitere Hilfsstoffe, wie beispielsweise Gelbildner, Verdickungsmittel, Duftstoffe, Konservierungsmittel o.dgl., auf.

Insbesondere variiert bei der erfindungsgemäßen Zubereitung die Konzentration des mindestens einen pharmazeutischen wirkstoffes und/oder des mindestens einen kosmetischen Wirkstoffes in der Zubereitung zwischen 0,001 Gew.% und 30 Gew.%, vorzugsweise zwischen 0,1 Gew.% und 15 Gew.%, jeweils bezogen auf die anwendungsfertige Zubereitung.

Eine besonders geeignete Ausführungsform der erfindungsgemäßen Zubereitung, die vorzugsweise für die topische Applikation verwendet wird, sieht vor, daß die Zubereitung desweiteren mindestens einen Alkohol, insbesondere Ethanol, Propanol-1 und/oder Propanol-2, enthält, wobei die Konzentration des mindestens einen Alkohols maximal 14 Gew.% beträgt und vorzugsweise zwischen 0,1 Gew.% und 8,5 Gew.% variiert, jeweils bezogen auf die anwendungsfertige Zubereitung. Diese Ausgestaltung der erfindungsgemäßen Zubereitung weist aufgrund des Alkoholgehaltes den zusätzlichen Vorteil auf, daß diese Ausführungsform der erfindungsgemäßen Zubereitung von vornherein schon steril ist, so daß die Anwendung von zusätzlichen Konservierungsmitteln entfallen kann. Außerdem konnte überraschend festgestellt werden, daß selbst bei hoher Empfindlichkeit des jeweiligen Anwenders gegenüber Alkohol keine Hautirritationen auftraten, sofern die Alkoholkonzentration in der erfindungsgemäßen Zubereitung auf maximal 14 Gew.% begrenzt wird. Dies wird darauf zurückgeführt, daß die erfindungsgemäße Zubereitung als wesentliche Inhaltsstoffe die Kombination von siliciumorganischer Verbindung mit. Phospholipid enthält, so daß durch diese hautheilende und hautschonende Kombination die negativen Hauteinflüsse des Alkohols ausgeschlossen werden. Selbst wenn man eine derartige Ausgestaltung der erfindungsgemäßen Zubereitung auf hochentzündliche Hautbereich topisch oder auf hochentzündliche Schleimhautbereiche aufträgt, konnten keine negativen Nebeneffekte, wie beispielsweise ein unangenehmes und schmerzhaftes Brennen, festgestellt werden.

Bezüglich des in der erfindungsgemäßen Zubereitung enthaltenen mindestens einen Wirkstoffs ist festzuhalten, daß sich zunächst die Auswahl des Wirkstoffes danach richtet, ob die erfindungsgemäße Zubereitung beim Menschen, beim Tier oder bei der Pflanze angewendet werden soll.

Für die menschliche Anwendung oder für die Anwendung beim Tier weist die erfindungsgemäße Zubereitung insbesondere einen solchen pharmazeutischen Wirkstoff auf, der aus der Gruppe ausgewählt, wird, die Lokalanästhetika, Antiallergika, Dermatika, Wirkstoffe gegen grippale Infekte und Erkältungskrankheiten, Wirkstoffe zur Behandlung von Neuropathien, Wirkstoffe zur Behandlung von Durchblutungsstörungen, Chemotherapeutika, Chinin, Antimykotika, Antibiotika, Thalidomid, Analgetika, Nicht-steroidale Antirheumatika, Leukotriene, Leukotrienhemmer, Antiandrogene, Kortikoide, Opiatrezeptor-Antagonisten, Blutgerinnung hemmende Stoffe, Thrombozytenaggregationshemmer, Histaminantagonisten, regulatorisch und enzymatisch wirkende Peptide und Proteine, Nukleinsäuren und Oligopeptide, Antidiabetika, Prostaglandine, Prostaglandinsynthesehemmer, Antiviral wirkende oder virostatisch wirkende Substanzen, Antimikrobiell-wirkende Substanzen, Wirkstoffe gegen Prione, Immunsupressiva, Hormone, Wirkstoffe zur Behandlung von Wunden und insbesondere chronischen Wunden, Vitamine, Pflanzenextrakte oder Auszüge aus Pflanzenextrakten, Psychopharmaka, den Schlaf beeinflussende Wirkstoffe, Analeptika, Allgemeinanästhetika, Muskelrelaxantien, Antiepileptika, Antiparkinsonmittel, Antiemetika, Ganglionär angreifende Substanzen, am Symphatikus angreifende Substanzen, am Parasamphatikus angreifende Substanzen, Calciumantagonisten, Herz-/Kreislaufmittel, Antiasthmatika, Antitussiva, Expektorantien, Hepatika, Diuretika, Choleretika, Desinfektionsmittel, Spurenelemente, Antiinfaktiva, Zytostatika, Antimetaboliten, Hormonanatgonisten, Immunmodulatoren und/oder Wirkstoffe gegen innere oder äußere Parasiten umfaßt.

Wird hingegen die erfindungsgemäße Zubereitung im Pflanzenbereich verwendet, so ist bei dieser speziellen Ausgestaltung der erfindungsgemäßen Zubereitung als wirkstoff ein Fungizid, ein Insektizid, ein Herbizid und/oder ein über Blatt- und/oder oberirdischen Pflanzenteilen aufnehmbares Düngemittel vorhanden.

Wie bereits zuvor wiederholt herausgestellt wurde, wird die erfindungsgemäße Zubereitung vorzugsweise auch topisch appliziert, wobei hierfür alle möglichen topischen Applikationsdarreichungsformen, so zum Beispiel eine topische Applikation als Gel, als Creme, als Spray, als Puder oder als Pflaster, denkbar sind.

Insbesondere weist die erfindungsgemäße Zubereitung für die topische Applikation eine flüssige bis pastöse Viskosität auf, wobei die Viskosität der Zubereitung vorzugsweise zwischen 0,4 mm²s⁻¹ (cSt) und 10.000 mm²s⁻¹ (cSt), insbesondere zwischen 0,6 mm²s⁻¹ (cSt) und 1.500 mm²s⁻¹ (cSt), variiert. Die in der vorliegenden Anmeldung verwendeten Viskositätsangaben werden alle bei 25 °C bestimmt. Diese flüssige bis pastöse Darreichungsform der erfindungsgemäßen Zubereitung-läßt sich einerseits sehr leicht auftragen und zieht andererseits sehr schnell ins Körperinnere ein, wodurch die Handhabung und die Effektivität der erfindungsgemäßen Zubereitung sichergestellt wird.

Insbesondere wird bei der zuvor beschriebenen topischen Applikation der erfindungsgemäßen Zubereitung die eine siliciumorganische Verbindung und das mindestens eine Phospholipid sowie ggf. ein Lösungsmittel, vorzugsweise ein Alkohol, bezüglich ihrer Massenverhältnisse derart aufeinander abgestimmt, daß nach Auftragen der Zubereitung auf die menschliche bzw. tierische Haut in einer Konzentration zwischen 0,05 g und 0,3 g, jeweils pro 100 cm² Haut, die so aufgetragene Zubereitung innerhalb einer Zeit von 1 Sekunde bis 60 Sekunden, vorzugsweise innerhalb einer Zeit von 3 Sekunden bis 20 Sekunden, keine sichtbaren Rückstände mehr auf der Haut verbleiben. Hierdurch wird dann verhindert, daß eine Beschmutzung der Kleidung und/oder ein unerwünschtes Abreiben bzw. Abstreifen der so topisch aufgetragenen Zubereitung herbeigeführt wird, wodurch eine leichte und reproduzierbare Anwendung der erfindungsgemäßen Zubereitung sichergestellt wird.

Zusätzlich zu den zuvor genannten Wirkstoffen oder anstelle der zuvor genannten Wirkstoffe weist eine weitere Ausführungsform der erfindungsgemäßen Zubereitung mindestens ein Vitamin und insbesondere Vitamin C, Vitamin E, Vitamin A und/oder Pro-Vitamin A auf, wobei diese Vitamine wahlweise als pharmazeutische und/oder kosmetische Wirkstoffe aufzufassen sind.

Wie bereits vorstehend wiederholt dargelegt ist, kann die erfindungsgemäße Zubereitung neben der topischen Applikation auch in jeder anderen Darreichungsform aufgemacht sein, wobei eine Gabe der erfindungsgemäßen Zubereitung als Kapsel, Tablette, Zäpfchen, Pflaster, Spray, Nebel, Gel, Puder und/oder Creme erfolgen kann. Neben diesen speziellen Darreichungsformen und/oder der besonders bevorzugten topischen Anwendung kann die erfindungsgemäße Zubereitung ebensogut buccal, nasal, oral, anal, per Injektion und/oder Infusion dargereicht werden.

Vorteilhafte Weiterbildungen der erfindungsgemäßen Zubereitung sind in den Unteransprüchen angegeben.

Bezüglich des im vorliegenden Text verwendeten Begriffs Wasser ist festzuhalten, daß dieser Begriff Wasser nicht nur Wasser selbst, d.h. destilliertes und/oder entionisiertes Wasser, sondern auch alle übrigen wäßrigen Systeme, so insbesondere Salz- oder Pufferlösungen, vorzugsweise Phosphat-, Citrat- und/oder Acetatpuffer, abdeckt.

Alkohol im Sinne der vorliegenden Beschreibung deckt alle aliphatischen Alkohole mit einer Kohlenstoffkettenlänge von 1 bis 30 sowie mit 1 bis 10 Hydroxyl-Gruppen ab, vorzugsweise jedoch die niedermolekularen einwertigen C₂-C₅-Alkohole sowie die entsprechenden zweiwertigen Glykole.

Die erfindungsgemäße Zubereitung wird nachfolgend anhand von Ausführungsbeispielen im Detail näher erläutert.

### Ausführungsbeispiel 1

### sprühbare Gelformulierung mit dem Wirkstoff Clotrimazol

Es wurde eine sprühbare Gelformulierung hergestellt, die die folgenden Inhaltsstoffe aufwies:

| | |
|---|---|
| Clotrimazol | 1 Gew.% |
| Propanol-2 | 8 Gew.% |
| Hexamethyldisiloxan | 78,8 Gew.% |
| Phospholipid | 10 Gew.% |
| (75 Gew.% Phosphatidylcholin, 25 Gew.% Ethanol) | |
| Pfefferminzöl | 0,2 Gew.% |
| 1,2-Propanidol | 2 Gew.% |

Zur Herstellung dieser Formulierung wurde der zuvor genannte kosmetische Wirkstoff vorgelegt und in dem Phospholipid, das 75 Gew.% Phosphatidylcholin und 25 Gew.% Ethanol aufwies, unter Zusatz etwa der Hälfte des zuvor ausgewiesenen Hexamethyldisiloxan unter Rühren bei Raumtemperatur gelöst. Nachdem eine klare Lösung resultierte, wurde unter Rühren bei Raumtemperatur die verbleibende Teilmenge Hexamethyldisiloxan zugesetzt.

Die so hergestellte sprühbare Formulierung, die gute Gel- und Filmbildungseigenschaften besaß und darüber hinaus noch stark benetzend war, wurde mit 2 Sprühstößen (jeweils 0,2 ml aus einem mechanischem Sprühsystem, Braunglasflasche verschlossen mit Mistette MARK II Sprühkopf) auf den Handrücken aus 20 cm Entfernung aufgetragen. Es bildete sich hierbei ein gleichmäßig verteilter homogener Film aus, der innerhalb von 60 Sekunden komplett ohne Hinterlassen eines Rückstandes in die Haut eingezogen bzw. verdunstet war.

Zur Bestimmung der Stabilität wurde der Wirkstoffgehalt direkt nach der Herstellung gemessen. Nach einer Lagerung von 60 Tagen bei 40 °C und einer Lagerung von 120 Tagen, ebenfalls bei 40 °C, konnte kein Verlust an Wirkstoff festgestellt werden. Auch konnte das Hauptabbauprodukt des Clotrimazols, d.h. das Carbinol, nach entsprechender Lagerung nicht detektiert werden.

Desweiteren wurden Lagerungsversuche durchgeführt, wobei jeweils eine Probe der zuvor genannten Sprühformulierung drei Monate bei 4 °C, drei Monate bei 25 °C und drei Monate bei 40 °C gelagert wurde. Bei keiner der gelagerten Proben trat eine Phasentrennung oder eine Kristallbildung auf. Alle gelagerten Proben waren, wie die Ausgangsprobe, dünnflüssig, leicht gelb gefärbt, transparent und sprühbar.

Die vorstehend beschriebene Sprühformulierung war darüber hinaus auf Fremdpartikel kontrollierbar, ließ sich steril filtrieren (0,2 µm Filter) und konnte durch einfaches Rühren ohne Homogenisieren und ohne zusätzliche Temperaturerhöhung bzw. Druckänderungen hergestellt werden.

Zum Nachweis der pharmazeutischen Anwendung der antimykotischen Sprühformulierung, die eine hohe Wirksamkeit bei allen Hautmykosen, so insbesondere bei Fußpilz, bei Vaginalmykosen und/oder Nagelmykosen, aufweist, die durch eine äußerliche Behandlung zugänglich sind, wurde die zuvor beschriebene und nach Ausführungsbeispiel 1 hergestellte Sprühformulierung im Vergleich zu einem herkömmlichen Handelsprodukt, das ebenfalls 1 Gew.% Clotrimazol als Wirkstoff in einer Formulierung, die zu mehr als 50 % aus Isopropanol zusammengesetzt war und ferner Macrogol 400 sowie Propylenglykol enthielt, bei 46 gesunden Probanden (zweimal 23 Probanden pro Gruppe) eine verblindete Verträglichkeitsstudie (dreimal täglich drei Sprühstöße à 0,2 ml auf ca. 100 cm² Haut im Bauchbereich) über einen Behandlungszeitraum von 10 Tagen durchgeführt.

Als Ergebnis dieser Studie ist festzuhalten, daß bei keinem der Probanden, die mit der Sprühformulierung gemäß Ausführungsbeispiel 1 behandelt wurden, eine Hautreaktion feststellbar war. Auch trat keine Austrocknung oder Unverträglichkeit im besprühten Bereich auf.

Sechs der Probanden, die mit dem herkömmlichen Produkt behandelt wurden, zeigten im behandelten Bereich der Bauchhaut im Vergleich zu der benachbarten unbehandelten Haut deutlich erkennbare Austrocknungen. Drei Probanden davon wiesen zusätzlich zur Hautaustrocknung noch Hautirritationen in Form von Hautrötungen und Pustelbildungen auf.

### Ausführungsbeispiel 2

Es wurden drei unterschiedliche, topisch applizierbare Formulierungen des Wirkstoffes Acetylsalicylsäure erstellt, wobei hier zur Herstellung dieser Formulierungen so vorgegangen wurde, wie dies vorstehend bei Ausführungsbeispiel 1 beschrieben ist. Die diesbezüglichen Formulierungen wiesen folgende Inhaltsstoffe auf:

| **Formulierung A** | |
|---|---|
| Acetylsalicylsäure | 12,1 Gew.% |
| Phospholipid | 28,2 Gew.% |
| Isopropanol | 38,1 Gew.% |
| Hexamethyldisiloxan | 21,6 Gew.% |

| **Formulierung C** | |
|---|---|
| Acetylsalicylsäure | 5,2 Gew.% |
| Phospholipid | 12,1 Gew.% |
| Isopropanol | 16,6 Gew.% |
| Hexamethyldisiloxan | 66,1 Gew.% |

| **Formulierung D** | |
|---|---|
| Acetylsalicylsäure | 3 Gew.% |
| Phospholipid | 7,2 Gew.% |
| Isopropanol | 9,8 Gew.% |
| Hexamethyldisiloxan | 80 Gew.% |

In den zuvor genannten Formulierungen A bis D wurde als Phospholipid eine Lösung von 75 Gew.% Phosphatidylcholin in 25 Gew.% Ethanol verwendet.

Die zuvor beschriebenen Formulierungen A bis D dienen zur topischen und lokalen epikutanen Anwendung, insbesondere im Bereich der Schmerzbekämpfung (Kopfschmerzen, Muskelschmerzen, Rheuma) und zur Blutverdünnung. Diese Formulierungen waren auch nach einer Lagerung von über drei Monaten bei einer Lagertemperatur von 40 °C stabil.

### Ausführungsbeispiel 3

Es wurde eine sprühbare und gelbildende Zubereitung zur Behandlung bei Sport- bzw. Unfallverletzungen, Weichteilrheumatismus, bei Schmerzen des Bewegungsapparates an Gelenken, Muskeln und/oder Sehnen, die durch unterschiedliche Unfälle, Überlastungen oder sonstigen Erkrankungen ausgelöst werden, aus folgenden Inhaltsstoffen erstellt:

| | |
|---|---|
| S-(+)-Flurbiprofen | 0,35 Gew.% |
| Hexamethyldisiloxan | 60,95 Gew.% |
| Phospholipid | 38,7 Gew.% |

### Ausführungsbeispiel 4

Es wurde eine Zubereitung zur Abfüllung in Hartgelatinekapseln zur systemischen Behandlung von sogenannten banalen Schmerzen (Kopf-, Gliederschmerzen, menstruationsbedingter Schmerzen), postoperativen Schmerzen sowie Schmerzen bedingt durch Erkrankungen aus dem rheumatischen Formenkreis aus folgenden Inhaltsstoffen erstellt:

| | |
|---|---|
| S-(+)-Flurbiprofen | 0,35 Gew.% |
| Hexamethyldisiloxan | 19,65 Gew.% |
| Phospholipid | 80 Gew.% |

Die zuvor genannte Zubereitung wurde in Hartgelatinekapseln (Größe 1) mit 450 µl der aufgeführten Formulierung befüllt.

### Ausführungsbeispiel 5

Es wurde eine Zubereitung zur Abfüllung in Hartgelatinekapseln zur systemischen Behandlung von sogenannten banalen Schmerzen (Kopf-, Gliederschmerzen, menstruationsbedingter Schmerzen), postoperativen Schmerzen sowie Schmerzen bedingt durch Erkrankungen aus dem rheumatischen Formenkreis aus folgenden Inhaltsstoffen erstellt:

| | |
|---|---|
| Dexibuprofen | 9,1 Gew.% |
| Hexamethyldisiloxan | 21,2 Gew.% |
| Phospholipid | 69,7 Gew.% |

Die zuvor genannte Zubereitung wurde in Hartgelatinekapseln (Größe 1) mit 450 µl der aufgeführten Formulierung befüllt.

### Ausführungsbeispiel 6

Es wurde eine Zubereitung zur Abfüllung in Hartgelatinekapseln zur systemischen Behandlung von sogenannten banalen Schmerzen (Kopf-, Gliederschmerzen, menstruationsbedingter Schmerzen), postoperativen Schmerzen sowie Schmerzen bedingt durch Erkrankungen aus dem rheumatischen Formenkreis aus folgenden Inhaltsstoffen erstellt :

| | |
|---|---|
| Dexibuprofen | 15 Gew.% |
| Hexamethyldisiloxan | 35 Gew.% |
| Phospholipid | 50 Gew.% |

Die zuvor genannte Zubereitung wurde in Hartgelatinekapseln (Größe 1) mit 450 µl der aufgeführten Formulierung befüllt.

Das in den Beispielen 4 bis 6 verwendete Phospholipid enthielt 76 ± 3 Gew.% Phosphatidylcholin, 3 ± 3 Gew.% Lysophosphatidylcholin, bis 8 Gew.% Phosphatidsäure, bis 4 Gew.% Phosphatidylethanolamin und maximal 9 Gew.% sonstiger phospholipidische und nicht-phospholipidische Bestandteile, wie insbesondere Öle, Fette und/oder Triglyceride.

### Ausführungsbeispiel 7

Es wurde eine flüssige Zubereitung zur Behandlung von Ekzemen, wie insbesondere endogene, toxisch-degenerative und seborr. Ekzeme, allerg. Kontaktekzeme, Stauungsekzeme, entzündliche und allerg. Hauterkrankungen, Psoriasis, Sonnenbrand, Alopezia Areata aus folgenden Inhaltsstoffen erstellt:

| | |
|---|---|
| Hydrocortison rein | 0,1 Gew.% |
| Hexamethyldisiloxan | 27,5 Gew.% |
| Propanol-2 reinst | 5 Gew.% |
| Phospholipid | 10,4 Gew.% |
| Emusifier 10 | 2 Gew.% |
| (Handelsprodukt der Firma Dow Corning) | |
| Wasser reinst | 45 Gew.% |
| Phosphatpuffer-2, | 10 Gew.% |
| pH 4,8, 10-fach | |

Die zuvor genannte wäßrige Zubereitung wird zwei- bis dreimal täglich mit ein bis zwei Sprühstößen (jeweils 0,2 ml) auf die erkrankte Hautfläche von 100 cm² topisch, epikutan aufgetragen bzw. aufgesprüht.

### Ausführungsbeispiel 8

Es wurde eine flüssige Zubereitung zur Behandlung von Ekzemen, wie insbesondere endogene, toxisch-degenerative und seborr. Ekzeme, allerg. Kontaktekzeme, Stauungsekzeme, entzündliche und allerg. Hauterkrankungen, Psoriasis, Sonnenbrand, Alopezia Areata aus folgenden Inhaltsstoffen erstellt:

| | |
|---|---|
| Hydrocortison rein | 0;1 Gew.% |
| Hexamethyldisiloxan | 65,4 Gew.% |
| Propanol-2 reinst | 5 Gew.% |
| Phospholipid | 8 Gew.% |
| Silmogen Carrier | 21,3 Gew.% |
| (Handelsprodukt der Firma Dow Corning) | |
| Pfefferminzöl | 0,15 Gew.% |

Die zuvor genannte wasserfreie sprühbare Zubereitung bildete beim Auftreffen auf die Haut spontan Liposome aus, deren mittlere Partikelgröße im Bereich von 194 nm lag.

### Ausführungsbeispiel 9

Es wurde eine flüssige Zubereitung zur Behandlung von Ekzemen, wie insbesondere endogene, toxisch-degenerative und seborr. Ekzeme, allerg. Kontaktekzeme, Stauungsekzeme, entzündliche und allerg. Hauterkrankungen, Psoriasis, Sonnenbrand, Alopezia Areata aus folgenden Inhaltsstoffen erstellt:

| | |
|---|---|
| Hydrocortison rein | 0,1 Gew.% |
| Hexamethyldisiloxan | 86,7 Gew.% |
| Propanol-2 reinst | 5 Gew.% |
| Phospholipid | 8 Gew.% |
| Pfefferminzöl | 0,2 Gew.% |

Die zuvor genannte wasserfreie sprühbare Zubereitung bildete beim Auftreffen auf die Haut spontan Liposome aus, deren mittlere Partikelgröße im Bereich von 205 nm lag.

### Ausführungsbeispiel 10

Es wurde eine flüssige, sprühbare Zubereitung zur Behandlung von Dermatomykosen, verursacht durch Dermatophyten, Hefen (z.B. Candida-Arten), Schimmelpilze und andere Pilze, mit entzündlichen bzw. ekzematösen Hauterscheinungen und/oder Juckreiz aus folgenden Inhaltsstoffen erstellt:

| | |
|---|---|
| Clotrimazol | 0,8 Gew.% |
| Propanol-2 | 11,2 Gew.% |
| Hexamethyldisiloxan | 77,8 Gew.% |
| Phospholipid | 10 Gew.% |
| Pfefferminzöl | 0,2 Gew.% |

Die zuvor genannte wasserfreie sprühbare Zubereitung bildete beim Auftreffen auf die Haut spontan Liposome aus, deren mittlere Partikelgröße im Bereich von 170 nm lag.

### Ausführungsbeispiel 11

Es wurde eine flüssige, sprühbare Zubereitung zur Behandlung bei Sport- bzw. Unfallverletzungen, Weichteilrheumatismus, bei Schmerzen des Bewegungsapparates an Gelenken, Muskeln und/oder Sehnen, die durch unterschiedliche Unfälle, Überlastungen oder sonstigen Erkrankungen ausgelöst werden, aus folgenden Inhaltsstoffen erstellt:

| | |
|---|---|
| Dexibuprofen | 8,3 Gew.% |
| Propanol-2 | 9 Gew.% |
| Hexamethyldisiloxan | 70,2 Gew.% |
| Phospholipid | 12,3 Gew.% |
| Pfefferminzöl | 0,2 Gew.% |

Die zuvor genannte wasserfreie sprühbare Zubereitung bildete beim Auftreffen auf die Haut spontan Liposome aus, deren mittlere Partikelgröße im Bereich von 105 nm lag.

### Ausführungsbeispiel 12

Es wurde eine flüssige, sprühbare Zubereitung zur Behandlung bei Sport- bzw. Unfallverletzungen, Weichteilrheumatismus, bei Schmerzen des Bewegungsapparates an Gelenken, Muskeln und/oder Sehnen, die durch unterschiedliche Unfälle, Überlastungen oder sonstigen Erkrankungen ausgelöst werden, aus folgenden Inhaltsstoffen erstellt:

| | |
|---|---|
| Ibuprofen | 8,3 Gew.% |
| Propanol-2 | 9 Gew.% |
| Hexamethyldisiloxan | 70,2 Gew.% |
| Phospholipid | 12,3 Gew.% |
| Pfefferminzöl | 0,2 Gew.% |

Die zuvor genannte wasserfreie sprühbare Zubereitung bildete beim Auftreffen auf die Haut spontan Liposome aus, deren mittlere Partikelgröße im Bereich von 135 nm lag.

### Ausführungsbeispiel 13

Es wurde eine flüssige, sprühbare Zubereitung zur Behandlung von endogenem Ekzem (atopische Dermatitis, Neurodermitis), degenerativem dyshidrotischem vulgärem Ekzem, Kontaktekzem, Neurodermitis aus folgenden Inhaltsstoffen erstellt:

| | |
|---|---|
| Prednisolon | 0,1 Gew.% |
| Propanol-2 | 5 Gew.% |
| Hexamethyldisiloxan | 86,7 Gew.% |
| Phospholipid | 8 Gew.% |
| Pfefferminzöl | 0,2 Gew.% |

Die zuvor genannte wasserfreie sprühbare Zubereitung bildete beim Auftreffen auf die Haut spontan Liposome aus, deren mittlere Partikelgröße im Bereich von 400 nm lag.

### Ausführungsbeispiel 14

Es wurde eine flüssige, sprühbare Zubereitung zur Behandlung von akuten und chronischen Ekzemen, atopischen Ekzemen (Neurodermitis), Psoriasis, Verbrennungen 1. Grades aus folgenden Inhaltsstoffen erstellt:

| | |
|---|---|
| Prednicarbat | 0,1 Gew.% |
| Propanol-2 | 5 Gew.% |
| Hexamethyldisiloxan | 86,7 Gew.% |
| Phospholipid | 8 Gew.% |
| Pfefferminzöl | 0,2 Gew.% |

Die zuvor genannte wasserfreie sprühbare Zubereitung bildete beim Auftreffen auf die Haut spontan Liposome aus, deren mittlere Partikelgröße im Bereich von 295 nm lag.

### Ausführungsbeispiel 15

Es wurde eine flüssige, sprühbare Zubereitung zur Behandlung bei Sport- bzw. Unfallverletzungen, Weichteilrheumatismus, bei Schmerzen des Bewegungsapparates an Gelenken, Muskeln und/oder Sehnen, die durch unterschiedliche Unfälle, Überlastungen oder sonstigen Erkrankungen ausgelöst werden, aus folgenden Inhaltsstoffen erstellt:

| | |
|---|---|
| Diclofenac | 5 Gew.% |
| Propanol-2 | 9 Gew.% |
| Hexamethyldisiloxan | 72,5 Gew.% |
| Phospholipid | 13,3 Gew.% |
| Pfefferminzöl | 0,2 Gew.% |

Die zuvor genannte wasserfreie sprühbare Zubereitung bildete beim Auftreffen auf die Haut spontan Liposome aus, deren mittlere Partikelgröße im Bereich von 50 bis 400 nm lag.

### Ausführungsbeispiel 16

Es wurde eine flüssige, sprühbare Zubereitung zur Behandlung von Pilzerkrankungen aus folgenden Inhaltsstoffen erstellt:

| | |
|---|---|
| Clotrimazol | 1 Gew.% |
| Propanol-2 | 11 Gew.% |
| Hexamethyldisiloxan | 79,8 Gew.% |
| Phospholipid | 8 Gew.% |
| Pfefferminzöl | 0,2 Gew.% |

Die zuvor genannte wasserfreie sprühbare Zubereitung bildete beim Auftreffen auf die Haut spontan Liposome aus, deren mittlere Partikelgröße im Bereich von 230 nm lag.

Von dieser Zubereitung gemäß Ausführungsbeispiel 16 wurde die Lagerstabilität gemessen. Nach einer extremen Lagerung von sechs Monaten bei 40 °C konnte der Clotrimazol-Gehalt der so gelagerten Zubereitung mit 99,9 %, bezogen auf die eingesetzte Ausgangskonzentration, festgestellt werden, so daß nur 0,1 % des ursprünglich eingesetzten Wirkstoffes zu Carbinol abgebaut wurde.

Das in den Beispielen 1 bis 3 sowie 7 bis 12 jeweils eingesetzten Phospholipide wiesen 25 Gew.% Ethanol und 75 Gew.% Phospholipid auf, das 76 ± 3 Gew.% Phosphatidylcholin, 3 ± 3 Gew.% Lysophosphatidylcholin, bis 8 Gew.% Phosphatidsäure, bis 4 Gew.% Phosphatidylethanolamin und maximal 9 Gew.% sonstiger phospholipidische und nicht-phospholipidische Bestandteile, wie insbesondere Öle, Fette und/oder Triglyceride, enthielt.

Das in den Beispielen 13 und 14 jeweils eingesetzte Phospholipid wies 15 Gew.% Isopropanol und 85 Gew.% Phospholipid auf, das 76 ± 3 Gew.% Phosphatidylcholin, 3 ± 3 Gew.% Lysophosphatidylcholin, bis 8 Gew.% Phosphatidsäure, bis 4 Gew.% Phosphatidylethanolamin und maximal 9 Gew.% sonstiger phospholipidische und nicht-phospholipidische Bestandteile, wie insbesondere Öle, Fette und/oder Triglyceride, enthielt.

Das in den Beispielen 15 und 16 jeweils eingesetzte Phospholipid wies 10 Gew.% Ethanol und 90 Gew.% Phospholipid auf, das 45 ± 5 Gew.% Phosphatidylcholin, 15 ± 3 Gew.% Phosphatidylethanolamin., 25 ± 4 Gew.% Phosphatidylinositol und maximal 15 Gew.% sonstiger phospholipidische und nicht-phospholipidische Bestandteile, wie insbesondere Öle, Fette und/oder Triglyceride, enthielt.

Zur Herstellung der in dem Beispiel 10 beschriebenen Zubereitungen wurde so vorgegangen, wie dies beim Ausführungsbeispiel 1 vorstehend dargelegt ist.

Um die in den Ausführungsbeispielen 2 bis 9 und 11 bis 16 beschriebenen Zubereitungen zu erstellen, wurde der jeweilige Wirkstoff vorgelegt und in einer Phase, die das Phospholipid sowie ggf. die weiteren Hilfsstoffe (Alkohol, Emulgator, Aromen etc.) enthielt, bei Raumtemperatur unter Rühren gelöst. Nachdem eine vollständige und transparente Lösung erstellt war, erfolgte der Zusatz des Siloxans unter weiterem Rühren bei Raumtemperatur.

Um die in den Ausführungsbeispielen 1 und 10 beschriebenen Zubereitung zu erstellen, wurde der jeweilige Wirkstoff vorgelegt und in einer Phase, die das Phospholipid, ggf. die weiteren Hilfsstoffe (Alkohol, Emulgator, Aromen etc.) und zwischen 10 Gew.% und 40 Gew.% der jeweiligen Gesamtmenge des eingesetzten Siloxans enthielt, bei Raumtemperatur unter Rühren gelöst. Nachdem eine vollständige und transparente Lösung erstellt war, erfolgte der Zusatz der Restmenge des Siloxans unter weiterem Rühren bei Raumtemperatur.

## Patentansprüche

1. Pharmazeutische und/oder kosmetische Zubereitung geeignet zur Anwendung beim Menschen, beim Tier oder bei Pflanzen mit mindestens einem pharmazeutischen und/oder kosmetischen Wirkstoff, mindestens einem Phospholipid sowie mit mindestens einer siliciumorganischen Verbindung auf der Basis eines Diorganosiloxanes, **dadurch gekennzeichnet, daß** die Zubereitung als siliciumorganische Verbindung Hexamethyldisiloxan enthält.

2. Zubereitung nach Anspruch 1, **dadurch gekennzeichnet, daß** die Zubereitung als Phospholipid ein aus natürlichen Ausgangsstoffen isoliertes Phospholipid aufweist.

3. Zubereitung nach Anspruch 1 oder 2, **dadurch gekennzeichnet, daß** die Zubereitung als Phospholipid mindestens ein an Phosphatidylcholin reiches Phospholipid aufweist und vorzugsweise ein solches Phospholipid enthält, das mehr als 70 Gew.% Phosphatidylcholin besitzt.

4. Zubereitung nach Anspruch 3, **dadurch gekennzeichnet, daß** das Phospholipid mindestens 80 Gew.% und vorzugsweise mehr als 90 Gew.% Phosphatidylcholin enthält.

5. Zubereitung nach einem der vorangehenden Ansprüche, **dadurch gekennzeichnet, daß** das Phospholipid ein hydriertes Phospholipid, vorzugsweise ein hydriertes Phosphatidylcholin, ist.

6. Zubereitung nach einem der vorangehenden Ansprüche, **dadurch gekennzeichnet, daß** die Zubereitung das Hexamethyldisiloxan in einer Konzentration zwischen 5 Gew.% und 96 Gew.%, bezogen auf das Gesamtgewicht der Zubereitung, und das mindestens eine Phospholipid in einer Konzentration zwischen 2 Gew.% und 30 Gew.%, bezogen auf das Gesamtgewicht der Zubereitung, aufweist.

7. Zubereitung nach Anspruch 6, **dadurch gekennzeichnet, daß** die Zubereitung zwischen 20-Gew.% und 85 Gew.% des Hexamethyldisiloxans und zwischen 5 Gew.% und 20 Gew.% des mindestens einen Phospholipids enthält.

8. Zubereitung nach einem der vorangehenden Ansprüche, **dadurch gekennzeichnet, daß** die Zubereitung zwischen 0 Gew.% und 5 Gew.% Wasser aufweist und daß das in der Zubereitung enthaltene Hexamethyldisiloxan und das Phospholipid derart miteinander vermischt sind, daß die Zubereitung bei Kontakt mit Wasser spontan Liposome und/oder Mizellen ausbildet.

9. Zubereitung nach Anspruch 8, **dadurch gekennzeichnet, daß** die durch Zugabe von Wasser sich spontan bildenden Liposome bzw. Mizellen eine mittlere Teilchengröße zwischen 50 nm und 4.000 nm, insbesondere zwischen 250 nm und 1.500 nm, aufweisen.

10. Zubereitung nach einem der vorangehenden Ansprüche, **dadurch gekennzeichnet, daß** die Zubereitung neben dem Hexamethyldisiloxan und dem mindestens einen Phospholipid desweiteren Wasser, Alkohol, Antioxidantien, nicht phospholipidische Emulgatoren, pharmazeutische Wirkstoffe, kosmetische Wirkstoffe und/oder weitere Hilfsstoffe aufweist.

11. Zubereitung nach Anspruch 10, **dadurch gekennzeichnet, daß** die Konzentration des mindestens einen pharmazeutischen Wirkstoffes und/oder des mindestens einen kosmetischen Wirkstoffes in der Zubereitung zwischen 0,001 Gew.% und 30 Gew.%, insbesondere zwischen 0,1 Gew.% und 15 Gew.%, bezogen auf die anwendungsfertige Zubereitung, variiert.

12. Zubereitung nach einem der vorangehenden Ansprüche, **dadurch gekennzeichnet, daß** die Zubereitung desweiteren mindestens einen Alkohol enthält, wobei die Konzentration des mindestens einen Alkohols maximal 14 Gew.% beträgt und vorzugsweise zwischen 0,1 Gew.% und 8,5 Gew.% variiert, jeweils bezogen auf die anwendungsfertige Zubereitung.

13. Zubereitung nach einem der vorangehenden Ansprüche, **dadurch gekennzeichnet, daß** die Zubereitung zur Anwendung beim Menschen oder Tier mindestens einen kosmetischen und/oder pharmazeutischen Wirkstoff aufweist, der aus der Gruppe ausgewählt ist, die Lokalanästhetika, Antiallergika, Dermatika, Wirkstoffe gegen grippale Infekte und Erkältungskrankheiten, Wirkstoffe zur Behandlung von Neuropathien, Wirkstoffe zur Behandlung von Durchblutungsstörungen, Chemotherapeutika, Chinin, Antimykotika, Antibiotika, Thalidomid, Analgetika, Nicht-steroidale Antirheumatika, Leukotriene, Leukotrienhemmer, Antiandrogene, Kortikoide, Opiatrezeptor-Antagonisten, Blutgerinnung hemmende Stoffe, Thrombozytenaggregationshemmer, Histaminantagonisten, regulatorisch und enzymatisch wirkende Peptide und Proteine, Nukleinsäuren und Oligopeptide, Antidiabetika, Prostaglandine, Prostaglandinsynthesehemmer, Antiviral wirkende oder virostatisch wirkende Substanzen, Antimikrobiell-wirkende Substanzen, Wirkstoffe gegen Prione, Immunsupressiva, Hormone, Wirkstoffe zur Behandlung von Wunden und insbesondere chronischen Wunden, Vitamine, Pflanzenextrakte oder Auszüge aus Pflanzenextrakten, Psychopharmaka, den Schlaf beeinflussende Wirkstoffe, Analeptika, Allgemeinanästhetika, Muskelrelaxantien, Antiepileptika, Antiparkinsonmittel, Antiemetika, Ganglionär angreifende Substanzen, am Symphatikus angreifende Substanzen, am Parasamphatikus angreifende Substanzen, Calciumantagonisten, Herz-/Kreislaufmittel, Antiasthmatika, Antitussiva, Expektorantien, Hepatika, Diuretika, Choleretika, Desinfektionsmittel, Spurenelemente, Antiinfaktiva, Zytostatika, Antimetaboliten, Hormonanatgonisten und/oder Immunmodulatoren umfaßt.

14. Zubereitung nach einem der Ansprüche 1 bis 12, **dadurch gekennzeichnet, daß** die Zubereitung zur Anwendung bei Pflanzen als Wirkstoff ein Fungizid, ein Insektizid, ein Herbizid und/oder ein über Blatt- und/oder oberirdischen Pflanzenteilen aufnehmbares Düngemittel aufweist.

15. Zubereitung nach einem der vorangehenden Ansprüche, **dadurch gekennzeichnet, daß** die Zubereitung eine flüssige bis pastöse Viskosität zur topischen Applikation aufweist, wobei die Viskosität der Zubereitung vorzugsweise zwischen 0,4 mm²s⁻¹ und 10.000 mm²s⁻¹, insbesondere zwischen 0,6 mm²s⁻¹ und 1.500 mm²s⁻¹, variiert.

16. Zubereitung nach Anspruch 15, **dadurch gekennzeichnet, daß** die Zubereitung das Hexamethyldisiloxan und das mindestens eine Phospholipid in einem solch abgestimmten Massenverhältnis aufweist, daß nach Auftragen der Zubereitung auf die menschliche bzw. tierische Haut in einer Konzentration zwischen 0,05 g und 0,3 g, jeweils pro 100 cm² Haut, innerhalb einer Zeit von 1 Sekunde bis 60 Sekunden, vorzugsweise innerhalb einer Zeit von 3 Sekunden bis 20 Sekunden, keine sichtbaren Rückstände der Zubereitung auf der Haut mehr verbleiben.

17. Zubereitung nach einem der vorangehenden Ansprüche, **dadurch gekennzeichnet, daß** die Zubereitung als pharmazeutischen und/oder kosmetischen Wirkstoff mindestens ein Vitamin aufweist.

18. Zubereitung nach Anspruch 17, **dadurch gekennzeichnet, daß** das Vitamin ein Vitamin C, Vitamin E, Vitamin A und/oder Pro-Vitamin A ist.

19. Zubereitung nach einem der vorangehenden Ansprüche, **dadurch gekennzeichnet, daß** die Zubereitung eine Darreichungsform besitzt, die eine Gabe der Zubereitung als Kapsel, Zäpfchen, Pflaster, Spray, Nebel, Gel, Puder und/oder Creme ermöglicht.

20. Zubereitung nach einem der vorangehenden Ansprüche, **dadurch gekennzeichnet, daß** sie zur topischen Applikation von pharmazeutischen und/oder kosmetischen Wirkstoffen, zur buccalen Applikation dieser Wirkstoffe und/oder zur oralen Applikation dieser Wirkstoffe aufgemacht ist.

## Claims

1. A pharmaceutical and/or cosmetic composition suitable for use on humans, animals or plants, comprising at least one pharmaceutical and/or cosmetic active ingredient, at least one phospholipid and at least one organosilicon compound based on diorganosiloxane, **characterized in that** the composition contains hexamethyldisiloxane as the organosilicon compound.

2. The composition according to claim 1, **characterized in that** the composition comprises as the phospholipid such a phospholipid, being isolated from natural starting materials.

3. The composition according to claim 1 or 2, **characterized in that** the composition comprises as the phospholipid at least one phospholipid having a high phosphatidylcholine content, preferably such a phospholipid having more than 70 % by weight phosphatidylcholine.

4. The composition according to claim 3, **characterized in that** the phospholipid contains at least 80 % by weight and preferably more than 90 % by weight phosphatidylcholine.

5. The composition according to one of the preceding claims, **characterized in that** the phospholipid is a hydrogenated phospholipid, preferably a hydrogenated phosphatidylcholine.

6. The composition according to one of the preceding claims, **characterized in that** the composition comprises the hexamethyldisiloxane in a concentration between 5 % by weight and 96 % by weight, based on the total weight of the composition, and the at least one phospholipid in a concentration between 2 % by weight and 30 % by weight, based on the total weight of the composition.

7. The composition according to claim 6, **characterized in that** the composition contains between 20 % by weight and 85 % by weight of the hexamethyldisiloxane and between 5 % by weight and 20 % by weight of the at least one phospholipid.

8. The composition according to one of the preceding claims, **characterized in that** the composition comprises between 0 % by weight and 5 % by weight water, and the hexamethyldisiloxane and the phospholipid contained in the composition are mixed together so that the composition spontaneously forms liposomes and/or micelles on coming in contact with water.

9. The composition according to claim 8, **characterized in that** the liposomes and/or micelles formed spontaneously by adding water have an average particle size between 50 nm and 4000 nm, in particular between 250 nm and 1500 nm.

10. The composition according to one of the preceding claims, **characterized in that** the composition also comprises water, alcohol, antioxidants, non-phospholipid emulsifiers, pharmaceutical active ingredients, cosmetic active ingredients and/or other auxiliary substances, in addition to the hexamethyldisiloxane and the at least one phospholipid.

11. The composition according to claim 10, **characterized in that** the concentration of the at least one pharmaceutical active ingredient and/or the at least one cosmetic active ingredient in the composition varies between 0,001 % by weight and 30 % by weight, in particular between 0,1 % by weight and 15 % by weight, based on the ready-to-use composition.

12. The composition according to one of the preceding claims, **characterized in that** the composition also contains at least one alcohol, where the concentration of the at least one alcohol amounts to a maximum of 14 % by weight and preferably varies between 0,1 % by weight and 8,5 % by weight, each based on the ready-to-use composition.

13. The composition according to one of the preceding claims, **characterized in that** the composition for use on humans or animals comprises at least one cosmetic and/or pharmaceutical active ingredient which is selected from the group consisting of local anesthetics, antiallergics, dermatologics, active ingredients to combat influenza infections and colds, active ingredients for treatment of neuropathies, active ingredients for treatment of circulation disorders, chemotherapeutics, quinine, antimycotics, antibiotics, thalidomide, analgesics, non-steroidal anti-inflammatory drugs, leukotrienes, leukotriene inhibitors, antiandrogens, corticosteroids, opiate receptor antagonists, blood coagulation inhibitors, platelet aggregation inhibitors, histamine antagonists, regulatory and enzymatic peptides and proteins, nucleic acids and oligopeptides, antidiabetics, prostaglandins, prostaglandin synthesis inhibitors, antiviral or virustatic substances, antimicrobial substances, active ingredients against prions, immunosuppressants, hormones, active ingredients for treatment of wounds and especially chronic wounds, vitamins, plant extracts or extracts of plant extracts, psycho-pharmaceuticals, active ingredients to influence sleep, analeptics, general anesthetics, muscle relaxants, antiepileptics, antiparkinsonian drugs, antiemetics, substances that act on the ganglia, substances that act on the sympathetic nervous system, substances that act on the parasympathetic nervous system, calcium antagonists, cardiovascular agents, antiasthmatics, antitussives, expectorants, hepatics, diuretics, choleretics, disinfectants, trace elements, anti-infectives, cytostatics, antimetabolites, hormone antagonists and/or immunomodulators.

14. The composition according to one of the claims 1 to 12, **characterized in that** the composition for use on plants contains as the active ingredient a fungicide, an insecticide, a herbicide and/or a fertilizer absorbable through leaf parts and/or above-ground plant parts.

15. The composition according to one of the preceding claims, **characterized in that** the composition has a liquid to pasty viscosity for topical application, whereby the viscosity of the composition preferably varies between 0,4 mm²s⁻¹ and 10.000 mm²s⁻¹, in particular between 0,6 mm²s⁻¹ and 1500 mm²s⁻¹.

16. The composition according to claim 15, **characterized in that** the composition comprises the hexamethyldisiloxane and the at least one phospholipid in such a coordinated weight ratio that after applying the composition to human or animal skin in a concentration between 0,05 and 0,3 g per 100 cm² skin, no visible residues of the composition remain on the skin within a period of 1 second to 60 seconds, preferably within a period of 3 seconds to 20 seconds.

17. The composition according to one of the preceding claims, **characterized in that** the composition comprises at least one vitamin as the pharmaceutical and/or cosmetic active ingredient.

18. The composition according to claim 17, **characterized in that** the vitamin is vitamin C, vitamin E, vitamin A and/or pro-vitamin A.

19. The composition according to one of the preceding claims, **characterized in that** the composition is in such a form of administration which permits administration of the composition as a capsule, suppository, plaster, spray, mist, gel, powder and/or cream.

20. The composition according to one of the preceding claims, **characterized in that** it is prepared for topical application of pharmaceutical and/or cosmetic active ingredients, for buccal application of these active ingredients and/or for oral administration of these active ingredients.

## Revendications

1. Préparation pharmaceutique et/ou cosmétique convenant à l'application à l'homme, à l'animal et aux végétaux ayant au moins une substance active pharmaceutique et/ou cosmétique, au moins un phospholipide ainsi qu'au moins un composé organosilicium à base d'un diorganosiloxane, **caractérisée en ce que** la préparation contient comme composé organosilicium de l'hexaméthyldisiloxane.

2. Préparation selon la revendication 1, **caractérisée en ce que** la préparation présente comme phospholipide un phospholipide isolé à partir de substances de départ naturelles.

3. Préparation selon la revendication 1 ou 2, **caractérisée en ce que** la préparation contient comme phospholipide au moins un phospholipide riche en phosphatidylcholine et de préférence un phospholipide de ce type qui possède plus de 70% en poids de phosphatidylcholine.

4. Préparation selon la revendication 3, **caractérisée en ce que** le phospholipide contient au moins 80% en poids et de préférence plus de 90% en poids de phosphatidylcholine.

5. Préparation selon l'une des revendications précédentes, **caractérisée en ce que** le phospholipide est un phospholipide hydrogéné, de préférence une phosphatidylcholine hydrogénée.

6. Préparation selon l'une des revendications précédentes, **caractérisée en ce que** la préparation présente l'hexaméthyldisiloxane dans une concentration entre 5% en poids et 96% en poids, sur base du poids total de la préparation, et le au moins un phospholipide dans une concentration entre 2% en poids et 30% en poids, sur base du poids total de la préparation.

7. Préparation selon la revendication 6, **caractérisée en ce que** la préparation contient entre 20% en poids et 85% en poids de l'hexaméthyldisiloxane et entre 5% en poids et 20% en poids du au moins un phospholipide.

8. Préparation selon l'une des revendications précédentes, **caractérisée en ce que** la préparation présente entre 0% en poids et 5% en poids d'eau, et **en ce que** l'hexaméthyldisiloxane contenu dans la préparation ainsi que le phospholipide sont mélangés l'un à l'autre de telle sorte que la préparation forme, au contact avec l'eau et spontanément, des liposomes et/ou des micelles.

9. Préparation selon la revendication 8, **caractérisée en ce que** les liposomes ou, selon le cas, micelles se formant spontanément par l'addition d'eau, présentent une grosseur moyenne de particules entre 50 nm et 4 000 nm, en particulier entre 250 nm et 1 500 nm.

10. Préparation selon l'une des revendications précédentes, **caractérisée en ce que** la préparation présente, à côté de l'hexaméthyldisiloxane et du au moins un phospholipide, encore de l'eau, un alcool, des antioxydants, des émulsionnants non phospholipidiques, des substances actives pharmaceutiques, des substances actives cosmétiques et/ou d'autres adjuvants.

11. Préparation selon la revendication 10, **caractérisée en ce que** la concentration de la au moins une substance active pharmaceutique et/ou de la au moins une substance active cosmétique dans la préparation varie de 0,001% en poids et 30% en poids, en particulier entre 0,1% en poids et 15% en poids, sur base de la préparation prête à l'emploi.

12. Préparation selon l'une des revendications précédentes, **caractérisée en ce que** la préparation contient en outre au moins un alcool, la concentration du au moins un alcool s'élevant à maximum 14% en poids et de préférence variant entre 0,1% en poids et 8,5% en poids, chaque fois sur base de la préparation prête à l'emploi.

13. Préparation selon l'une des revendications précédentes, **caractérisée en ce que** la préparation pour application à l'homme ou à l'animal présente au moins une substance active cosmétique et/ou pharmaceutique sélectionnée parmi le groupe englobant les anesthésiques locaux, les antiallergiques, les dermatiques, les substances contre les infections grippales et les maladies du refroidissement, les substances de traitement des maladies neurologiques, des substances de traitement des troubles de l'irrigation, des produits de chimiothérapie, de la chinine, des antimyotiques, des antibiotiques, un thalidomide, des analgésiques, des antirhumatiques non stéroïdaux, des leucotriènes, des inhibiteurs de leucotriène, des antiandrogènes, des corticoïdes, des antagonistes du récepteur opiate, des substances inhibant la coagulation du sang, des inhibiteurs d'agrégation des thrombocytes, des antagonistes d'histamines, des peptides à action régulatrice et enzymatique et des protéines, des acides nucléiques et des oligopeptides, des antidiabétiques, des prostaglandines, des inhibiteurs de synthèse de prostaglandine, des substances à action antivirale ou virostatique, des substances à action antimicrobienne, des substances actives contre les priones, des immunosuppressifs, des hormones, des substances actives pour le traitement des plaies et en particulier les plaies chroniques, des vitamines, des extraits de plantes ou des extraits d'extraits de plantes, des psychopharmaka, des substances actives influençant le sommeil, des analeptiques, des anesthésiques généraux, des relaxants musculaires, des antiépileptiques, des produits anti-Parkinson, des antiémétiques, des substances attaquant les ganglions, des substances attaquant le domaine sympathique, des substances attaquant le domaine parasympathique, des antagonistes du calcium, des substances pour le coeur/la circulation, des antiasthmatiques, des antitussifs, des expectorants, des hépatiques, des diurétiques, des cholérétiques, des agents de désinfection, des éléments en traces, des anti-infections, des cytostatiques, des antimétabolites, des antagonistes hormonaux et/ou des immunodulateurs.

14. Préparation selon l'une des revendications 1 à 12, **caractérisée en ce que** la préparation d'application aux végétaux présente comme substance active un fongicide, un insecticide, un herbicide et/ou un engrais absorbable par les feuilles et/ou les parties aériennes des plantes.

15. Préparation selon l'une des revendications précédentes, **caractérisée en ce que** la préparation présente une viscosité de liquide à pâteuse d'application topique, la viscosité de la préparation variant de préférence entre 0,4 mm² s⁻¹ et 10 000 mm²s⁻¹, en particulier entre 0,6 mm² s⁻¹ et 1 500 mm² s⁻¹.

16. Préparation selon la revendication 15, **caractérisée en ce que** la préparation présente l'hexaméthyldisiloxane et le au moins un phospholipide dans un rapport de masse déterminé de telle manière qu'après l'application de la préparation sur la peau humaine et/ou animale dans une concentration entre 0,05 g et 0,3 g, chaque fois pour 100 cm² de peau, en l'espace d'une durée d'1 seconde à 60 secondes, de préférence en l'espace d'une durée de 3 secondes à 20 secondes, il ne reste plus aucun résidu visible de la préparation sur la peau.

17. Préparation selon l'une des revendications précédentes, **caractérisée en ce que** la préparation présente comme substance active pharmaceutique et/ou cosmétique, au moins une vitamine.

18. Préparation selon la revendication 17, **caractérisée en ce que** la vitamine est une vitamine C, une vitamine E, une vitamine A et/ou une provitamine A.

19. Préparation selon l'une des revendications précédentes, **caractérisée en ce que** la préparation possède une posologie qui permet une administration de la préparation sous forme de capsule, de goutte scorifiée, des pansement, de spray, de brume, de gel, de poudre et/ou de crème.

20. Préparation selon l'une des revendications précédentes, **caractérisée en ce qu'**elle se présente pour une application topique de substances actives pharmaceutiques et/ou cosmétiques, pour une application buccale de ces substances actives et/ou pour une application orale de ces substances actives.
